(19) Europäisches Patentamt
European Patent Office
Office européen des brevets

(11) EP 4 741 509 A1

(12) **EUROPEAN PATENT APPLICATION**
published in accordance with Art. 153(4) EPC

(43) Date of publication:
**13.05.2026 Bulletin 2026/20**

(21) Application number: **25816420.1**

(22) Date of filing: **29.05.2025**

(51) International Patent Classification (IPC):
***C12P 7/52** (2006.01)* ***C07C 51/09** (2006.01)*
***C07C 51/377** (2006.01)* ***C07C 57/04** (2006.01)*

(52) Cooperative Patent Classification (CPC):
**C07C 51/09; C07C 51/377; C07C 57/04; C12P 7/52**

(86) International application number:
**PCT/KR2025/007352**

(87) International publication number:
**WO 2025/249930 (04.12.2025 Gazette 2025/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **31.05.2024 KR 20240071883**

(71) Applicant: **LG Chem, Ltd.**
**Seoul 07336 (KR)**

(72) Inventors:
- **NAM, Gibok**
  **Daejeon 34122 (KR)**
- **JEONG, Dae Youn**
  **Daejeon 34122 (KR)**
- **LEE, Jungyong**
  **Daejeon 34122 (KR)**
- **LEE, Hee Cheon**
  **Daejeon 34122 (KR)**
- **CHOE, Jae Hoon**
  **Daejeon 34122 (KR)**
- **BANG, Yongju**
  **Daejeon 34122 (KR)**

(74) Representative: **Plasseraud IP**
**104 Rue de Richelieu**
**CS92104**
**75080 Paris Cedex 02 (FR)**

# (54) METHOD FOR PREPARATION OF BRANCHED 3-HYDROXYPROPIONIC ACID PREPOLYMER AND ACRYLIC ACID

(57) The present invention relates to a method for preparing a fermentation broth by fermenting a strain capable of producing 3-hydroxypropionic acid in the presence of a polyol, and preparing a branched 3-hydroxypropionic acid prepolymer by polymerizing in the presence of the polyol, and a method for preparing bio-based acrylic acid by pyrolyzing the branched 3-hydroxypropionic acid prepolymer.

EP 4 741 509 A1

## Description

## FIELD OF THE INVENTION

CROSS-REFERENCE TO RELATED APPLICATION(S)

This application claims the benefit of Korean Patent Application No. 10-2024-0071883 filed on May 31, 2024, and Korean Patent Application No. 10-2025-0070312 filed on May 29, 2025 with the Korean Intellectual Property Office, the disclosure of which is incorporated herein by reference in its entirety.

The present invention relates to a method for preparing a high-purity branched 3-hydroxypropionic acid prepolymer and bio-based acrylic acid.

## BACKGROUND OF THE INVENTION

Organic acids are commercially important chemicals with various uses in the food, cosmetic, pharmaceutical, and polymer industries. Representative organic acids include lactic acid, 3-hydroxypropionic acid (3-HP), and the like, and among them, 3-hydroxypropionic acid (3-HP) is a substance that can be utilized as a raw material for the preparation of acrylic acid, 1,3-propanediol, acrylamide, malonic acid, and biopolymers.

The production of organic acids is largely carried out by two methods, a chemical method and a biological method, but the chemical method has been pointed out as being non-eco-friendly due to the high cost of starting materials and the generation of toxic substances during the production process, and thus, eco-friendly bio-processes are gaining attention.

In the preparation of organic acids by microbial fermentation, since other by-products are also produced along with organic acids such as 3-hydroxypropionic acid during the process of fermenting microorganisms, purification processes such as electrodialysis and ion exchange resin are required to extract and separate the organic acids from the fermentation broth, and there is a problem that the process of performing these purification processes is long and the process cost is high. Accordingly, there is a need for a method to efficiently convert low-purity organic acids such as 3-hydroxypropionic acid containing by-products into raw materials for biopolymers and the like.

## SUMMARY OF THE INVENTION

### TECHNICAL PROBLEM

The present invention relates to a method for preparing a high-molecular-weight and high-purity branched prepolymer from low-purity, unpurified 3-hydroxypropionic acid efficiently and at a low process cost, and preparing bio-based acrylic acid by pyrolyzing it.

### TECHNICAL SOLUTION

In the present specification, there is provided a method for preparing branched 3-hydroxypropionic acid prepolymer, comprising a step of fermenting a strain capable of producing 3-hydroxypropionic acid in the presence of a polyol to prepare a fermentation broth comprising the polyol and 3-hydroxypropionic acid, and a step of polymerizing the 3-hydroxypropionic acid in the presence of the polyol to prepare a branched 3-hydroxypropionic acid prepolymer.

In addition, in the present specification, there is provided a method for preparing acrylic acid, comprising a step of fermenting a strain capable of producing 3-hydroxypropionic acid in the presence of a polyol to prepare a fermentation broth comprising the polyol and 3-hydroxypropionic acid, a step of polymerizing the 3-hydroxypropionic acid in the presence of the polyol to prepare a branched 3-hydroxypropionic acid prepolymer, and a step of pyrolyzing the branched 3-hydroxypropionic acid prepolymer to prepare acrylic acid.

Hereinafter, the method for preparing branched 3-hydroxypropionic acid prepolymer and acrylic acid according to a specific embodiment of the present invention will be described in more detail.

The terminology used in this specification is used only to describe exemplary embodiments and is not intended to limit the present invention. A singular expression includes a plural expression unless the context clearly indicates otherwise. In this specification, it should be understood that terms such as "comprising," "including," or "having" are intended to designate the presence of stated features, numbers, steps, components, or combinations thereof, and do not preclude the possibility of the presence or addition of one or more other features, numbers, steps, components, or combinations thereof.

Since the present invention may be variously modified and have various forms, specific embodiments will be illustrated and described in detail below. However, this is not intended to limit the present invention to the specific disclosed

forms, and it should be understood to include all modifications, equivalents, and substitutes included in the spirit and technical scope of the present invention.

In addition, unless the steps constituting the preparation method described in this specification are specified as being sequential or continuous, or there is another special class, one step constituting one preparation method and another step are not interpreted as being limited to the order described in the specification. Therefore, the order of the constituent steps of the preparation method can be changed within a range that can be easily understood by those skilled in the art, and in this case, the attendant changes obvious to those skilled in the art are included in the scope of the present invention.

In the present specification, a prepolymer may be a polymer with a low degree of polymerization in which the polymerization reaction is stopped at an intermediate stage, and a 3-hydroxypropionic acid prepolymer may be an oligomerized product obtained by polymerizing 3-hydroxypropionic acid, and may have a meaning including a 3-hydroxypropionic acid oligomer.

In the present specification, an alkali metal may have a meaning including both alkali metals and alkaline earth metals.

In addition, in the present specification, the weight average molecular weight (Mw) and the number average molecular weight (Mn) mean the molecular weight in terms of polystyrene (unit: Da (Dalton)) measured by a Gel Permeation Chromatography (GPC) method. In the process of measuring the weight average molecular weight in terms of polystyrene measured by the GPC method, a commonly known analysis device, a detector such as a Refractive Index Detector, and an analysis column can be used, and commonly applied temperature conditions, solvent, and flow rate can be applied. As a specific example of the measurement conditions, a temperature of 30°C, a chloroform solvent (Chloroform), and a flow rate of 1 mL/min can be mentioned. To give a specific example of the measurement conditions, using a Polymer Laboratories PLgel MIX-B 300mm length column and a Waters PL-GPC220 instrument, the evaluation temperature is 160°C, 1,2,4-trichlorobenzene was used as a solvent, the flow rate was 1 mL/min, the sample was prepared at a concentration of 10 mg/10 mL and then supplied in an amount of 200 μL, and the values of Mw and Mn can be respectively obtained using a calibration curve formed using polystyrene standards. Nine types of polystyrene standards with molecular weights of 2,000 / 10,000 / 30,000 / 70,000 / 200,000 / 700,000 / 2,000,000 / 4,000,000 / 10,000,000 were used.

In the present specification, the term "substituted or unsubstituted" means substituted or unsubstituted with one or more substituents selected from the group consisting of deuterium; a halogen group; a nitrile group; a nitro group; a hydroxyl group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; or a heterocyclic group containing one or more of N, O, and S atoms, or substituted or unsubstituted with a substituent in which two or more of the exemplified substituents are linked. For example, "a substituent in which two or more substituents are linked" may be a biphenyl group. That is, a biphenyl group may be an aryl group, or it may be interpreted as a substituent in which two phenyl groups are linked.

In the present specification, the number of carbon atoms of the carbonyl group is not particularly limited, but it is preferably 1 to 40. Specifically, it may be a compound with the following structure, but is not limited thereto.

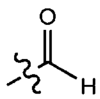 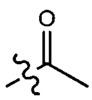 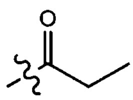 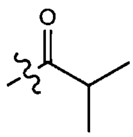

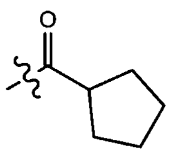 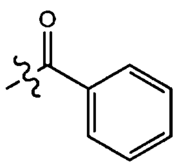 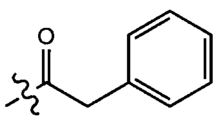

In the present specification, in the ester group, the oxygen of the ester group may be substituted with a C1-25 linear, branched, or cyclic alkyl group or a C6-25 aryl group. Specifically, it may be a compound of the following structural formula, but is not limited thereto.

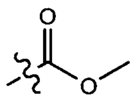 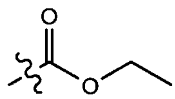 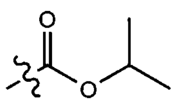 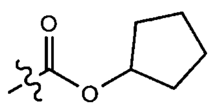 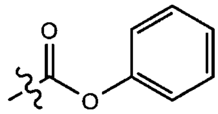

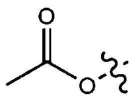 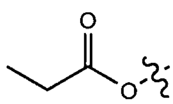 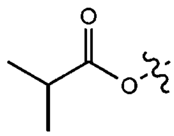 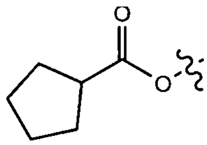 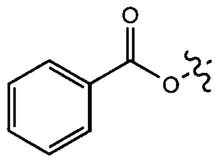

In the present specification, the number of carbon atoms of the imide group is not particularly limited, but it is preferably 1 to 25. Specifically, it may be a compound with the following structure, but is not limited thereto.

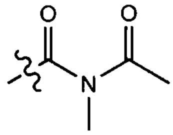 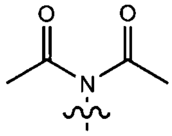 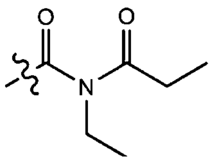

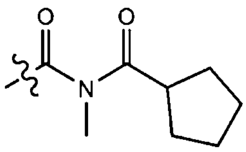 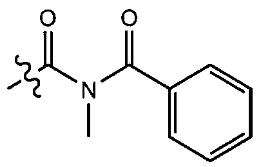

In the present specification, the silyl group specifically includes, but is not limited to, a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like.

In the present specification, the boron group specifically includes, but is not limited to, a trimethylboron group, a triethylboron group, a t-butyldimethylboron group, a triphenylboron group, a phenylboron group, and the like.

In the present specification, examples of the halogen group include fluorine, chlorine, bromine, or iodine.

In the present specification, the alkyl group may be linear or branched, and the number of carbon atoms is not particularly limited but is preferably 1 to 40. According to one embodiment, the number of carbon atoms of the alkyl group is 1 to 20. According to another embodiment, the number of carbon atoms of the alkyl group is 1 to 10. According to another embodiment, the number of carbon atoms of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethylpropyl, isohexyl, 4-methylhexyl, 5-methylhexyl, and the like, but are not limited thereto.

In the present specification, the alkenyl group may be linear or branched, and the number of carbon atoms is not particularly limited, but is preferably 2 to 40. According to one embodiment, the number of carbon atoms of the alkenyl group is 2 to 20. According to another embodiment, the number of carbon atoms of the alkenyl group is 2 to 10. According to another embodiment, the number of carbon atoms of the alkenyl group is 2 to 6. Specific examples include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present specification, the cycloalkyl group is not particularly limited, but it is preferably C3-60, and according to one embodiment, the number of carbon atoms of the cycloalkyl group is 3 to 30. According to another embodiment, the number of carbon atoms of the cycloalkyl group is 3 to 20. According to another embodiment, the number of carbon atoms of the cycloalkyl group is 3 to 6. Specifically, examples thereof include, but are not limited to cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present specification, the aryl group is not particularly limited, but it is preferably C6-60, and may be a monocyclic aryl group or a polycyclic aryl group. According to one embodiment, the number of carbon atoms of the aryl group is 6 to 30. According to one embodiment, the number of carbon atoms of the aryl group is 6 to 20. The monocyclic aryl group may be a phenyl group, a biphenyl group, a terphenyl group, or the like, but is not limited thereto. The polycyclic aryl group may be a naphthyl group, an anthracenyl group, a phenanthryl group, a pyrenyl group, a perylenyl group, a chrysenyl group, a fluorenyl group, or the like, but is not limited thereto.

In the present specification, the fluorenyl group may be substituted, and two substituents may be bonded to each other to form a spiro structure. When the fluorenyl group is substituted, it may be

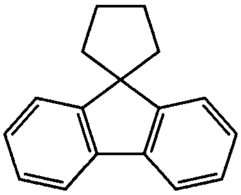 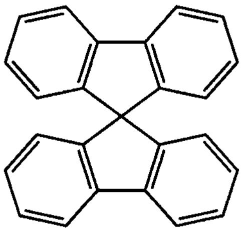 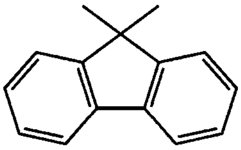 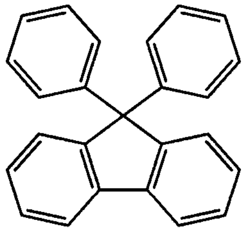 ,

and the like. However, it is not limited thereto.

In the present specification, a heteroaryl group contains one or more of O, N, Si, and S as heteroatoms, is a heterocyclic group having aromaticity, and the number of carbon atoms is not particularly limited, but is preferably 2 to 60. Examples of the heteroaryl group include a thiophene group, a furan group, a pyrrole group, an imidazole group, a thiazole group, an oxazole group, an oxadiazole group, a triazole group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzimidazole group, a benzothiazole group, a benzocarbazole group, a benzothiophene group, a dibenzothiophene group, a benzofuranyl group, a phenanthroline group, a thiazolyl group, an isoxazolyl group, an oxadiazolyl group, a thiadiazolyl group, a benzothiazolyl group, a phenothiazinyl group, and a dibenzofuranyl group, but are not limited thereto.

In the present specification, the aryl in the aralkyl group, aralkenyl group, alkylaryl group, and arylamine group is the same as the examples of the aryl group described above. In the present specification, the alkyl in the aralkyl group, alkylaryl group, and alkylamine group is the same as the examples of the alkyl group described above. In the present specification, the description regarding the heterocyclic group described above may be applied to the heteroaryl in the heteroarylamine. In the present specification, the alkenyl in the aralkenyl group is the same as the examples of the alkenyl group described above. In the present specification, the description regarding the aryl group described above may be applied to arylene, except that it is a divalent group. In the present specification, the description regarding the heterocyclic group described above may be applied to heteroarylene, except that it is a divalent group. In the present specification, the description regarding the aryl group or cycloalkyl group described above may be applied to the hydrocarbon ring, except that it is not a monovalent group and is formed by bonding two substituents. In the present specification, the description regarding the heterocyclic group described above may be applied to the heterocycle, except that it is not a monovalent group and is formed by bonding two substituents.

According to one embodiment of the present invention, a method for preparing branched 3-hydroxypropionic acid prepolymer is provided, comprising a step of fermenting a strain capable of producing 3-hydroxypropionic acid in the presence of a polyol to prepare a fermentation broth comprising the polyol and 3-hydroxypropionic acid, and a step of polymerizing the 3-hydroxypropionic acid in the presence of the polyol to prepare a branched 3-hydroxypropionic acid prepolymer.

When 3-hydroxypropionic acid is prepared by a biological method, other by-products such as inorganic impurities and organic impurities are also generated, and in the case of 3-hydroxypropionic acid, there is a problem that separation through distillation, which is used in the purification of other organic acids, is difficult because the difference in boiling point with water is almost nonexistent and there is a high possibility of pyrolysis. Accordingly, conventionally, complex processes such as electrodialysis, ion exchange resin, and SMB (Simulated Moving Bed) were performed to purify 3-hydroxypropionic acid, and these processes have the problem of high equipment costs and process operating costs.

In this regard, the present inventors have confirmed that when 3-hydroxypropionic acid is polymerized using a polyol used in the fermentation of a strain capable of producing 3-hydroxypropionic acid, a branched 3-hydroxypropionic acid prepolymer with a high molecular weight can be prepared, and that such a prepolymer exhibits hydrophobicity, making it easy to remove inorganic and organic impurities when washing it in a washing process described below.

In addition, since the purification process is hardly carried out in order to use the polyol used in the fermentation of

the strain capable of producing 3-hydroxypropionic acid in the polymerization process (for example, processes such as electrodialysis, ion exchange resin, SMB, etc., which have high equipment and process running costs, are not carried out), it is efficient, the process cost can be significantly reduced compared to the conventional art, and finally, the 3-hydroxypropionic acid prepolymer can be prepared in high purity and high yield.

In particular, in the purification process, loss of the purification target (3-hydroxypropionic acid) occurs at each step, and as the purification process becomes more diverse, there is a problem that a lot of loss finally occurs, but in the case of the one embodiment, since there are few purification steps where loss occurs, the 3-hydroxypropionic acid prepolymer can be prepared with a high yield.

In addition, the one embodiment can improve the hydrophobicity of the 3-hydroxypropionic acid prepolymer by controlling the content of the polyol during polymerization, the polymerization temperature, the molecular weight of the prepolymer, the content of vinyl groups at the terminal of the prepolymer, etc., in order to easily remove impurities in the process of washing the 3-hydroxypropionic acid prepolymer.

Furthermore, the 3-hydroxypropionic acid prepolymer can be pyrolyzed as described below to prepare high-value bio-based acrylic acid, and this bio-based acrylic acid has the advantage of being easily separable through distillation.

The method for preparing branched 3-hydroxypropionic acid prepolymer according to the one embodiment comprises a step of fermenting a strain capable of producing 3-hydroxypropionic acid in the presence of a polyol to prepare a fermentation broth comprising the polyol and 3-hydroxypropionic acid.

A microorganism such as a strain capable of producing 3-hydroxypropionic acid can produce a 3-hydroxypropionic acid fermentation broth by fermenting low-molecular-weight sugars. Such a microorganism may be a natural microorganism or an engineered microorganism, and the microorganism may be, for example, a bacterium, for example, a cellulose-degrading bacterium, a fungus, for example, a yeast, a plant or a protist, for example, algae, protozoa or a fungus-like protist, for example, a slime mold, and if the organism does not cause a rejection reaction, a mixture of organisms may be used.

For example, the strain capable of producing 3-hydroxypropionic acid may be one comprising a gene encoding one or more selected from the group consisting of glycerol dehydratase and aldehyde dehydrogenase or the two proteins. In one example, the 3-hydroxypropionic acid-producing strain may further comprise a gene (gdrAB) encoding a glycerol dehydratase reactivating enzyme (GdrAB). In one example, the 3-hydroxypropionic acid-producing strain may further be a strain capable of biosynthesizing vitamin $B_{12}$.

The glycerol dehydratase may be one encoded by the dhaB (GenBank accession no. U30903.1) gene, but is not limited thereto. The dhaB gene may be an enzyme derived from Klebsiella pneumonia, but is not limited thereto. The gene encoding the glycerol dehydratase may comprise a gene encoding dhaB1, dhaB2, and/or dhaB3. The glycerol dehydratase protein and the gene encoding it may include mutations in the gene and/or amino acid sequence within a range that maintains the enzyme activity of decomposing glycerol into 3-hydroxypropanal (3-HPA) and water ($H_2O$).

The gene (aldH) encoding aldehyde dehydrogenase (ALDH) may be, for example, the aldH (GenBank Accession no. U00096.3; EaldH) gene derived from Escherichia coli or the E. coli K12 MG1655 cell line, the puuC gene derived from K. pneumonia, and/or the KGSADH gene derived from Azospirillum brasilense, but is not limited thereto. The aldehyde dehydrogenase protein and the gene encoding it may include mutations in the gene and/or amino acid sequence within a range that maintains the activity for producing 3-hydroxypropionic acid from 3-hydroxypropanal.

The medium for the production of the fermentation broth may be selected without limitation within the scope of the purpose for producing 3-hydroxypropionic acid. In one example, the medium may be one comprising a polyol such as glycerol as a carbon source. In another example, the medium may be crude glycerol and/or pretreated crude glycerol, but is not limited thereto. In one example, the production medium may further comprise vitamin $B_{12}$.

In the step of fermenting the strain capable of producing 3-hydroxypropionic acid in the presence of the polyol to prepare the fermentation broth comprising the polyol and 3-hydroxypropionic acid, the concentration of 3-hydroxypropionic acid contained in the fermentation broth may be 1 g/L or more and 200 g/L or less, 10 g/L or more and 150 g/L or less, 30 g/L or more and 130 g/L or less, or 40 g/L or more and 100 g/L or less.

In addition, the fermentation may be neutral fermentation, for example, the pH during fermentation may be maintained in the range of 6.0 or more and 8.0 or less, 6.5 or more and 7.5 or less, or 6.5 or more and 7.5 or less, but is not limited thereto. The pH range may be appropriately adjusted as needed. For the neutral fermentation, the alkali metal salt may be added. The alkali metal salt may be one comprising $Mg^{2+}$, $Ca^{2+}$, or a mixture thereof. In addition, the alkali metal salt may be $Ca(OH)_2$ or $Mg(OH)_2$, but is not limited thereto.

In addition, the method for preparing branched 3-hydroxypropionic acid prepolymer according to the one embodiment may further comprise a step of separating the strain capable of producing 3-hydroxypropionic acid; and/or a step of generating and separating metal salt crystals by adding an acid to the fermentation broth comprising the polyol and 3-hydroxypropionic acid, after the step of fermenting a strain capable of producing 3-hydroxypropionic acid in the presence of a polyol to prepare a fermentation broth comprising the polyol and 3-hydroxypropionic acid.

Specifically, after the preparation of the fermentation broth, the strain capable of producing 3-hydroxypropionic

acid may be separated from the fermentation broth, and the strain separation may be performed by selecting a method known in the art without limitation within the scope of the purpose of strain removal. In one example, the separation of the strain may be performed by carrying out centrifugation.

In addition, after the fermentation broth preparation step or the strain removal step, an acid may be added to the fermentation broth to generate and separate metal salt crystals, and the metal salt crystals prepared by the acid addition may be $CaSO_4$(s) or $MgSO_4$(s), but are not limited thereto. At this time, the acid is not particularly limited as long as it is an acid that can control the pH of the fermentation broth to 5 or less, but may be, for example, one selected from the group consisting of sulfuric acid, hydrochloric acid, phosphoric acid, carbonic acid, and nitric acid. In addition, by adding the acid to the fermentation broth, the pH of the fermentation broth can be controlled to 5.0 or less, 4.0 or less, 3.0 or less, 2.0 or less, 1.0 or less, or 0.5 or less.

In addition, after the fermentation broth preparation step, the strain removal step, or the metal salt crystal removal step, an acid or an acid-based catalyst may be additionally added to protonate the 3-hydroxypropionic acid, and this protonation may also be achieved with the acid added in the metal salt crystal removal step without adding additional acid.

The method for preparing branched 3-hydroxypropionic acid prepolymer according to the one embodiment comprises a step of polymerizing the 3-hydroxypropionic acid in the presence of the polyol to prepare a branched 3-hydroxypropionic acid prepolymer.

The polyol may be a carbon source used for preparing the 3-hydroxypropionic acid fermentation broth, a by-product generated after fermenting the strain capable of producing 3-hydroxypropionic acid, or a mixture thereof. The method for preparing branched 3-hydroxypropionic acid prepolymer according to the one embodiment can prepare a branched prepolymer by polymerizing 3-hydroxypropionic acid in the presence of a polyol that was used in or generated during fermentation, without separately adding a polyol during the reaction in the 3-hydroxypropionic acid polymerization.

In addition, the preparation method according to the one embodiment does not proceed with a separate purification process immediately after the fermentation process in order to reuse the polyol for polymerization, so the process is efficient and the process cost is significantly reduced compared to the conventional art, and the loss of the purification target (3-hydroxypropionic acid) due to the purification process can be prevented.

Furthermore, polyols and inorganic by-products, etc., are easily removed through the subsequent washing process of the prepolymer, so that a high-purity and high-yield branched 3-hydroxypropionic acid prepolymer can be prepared.

In addition, since the 3-hydroxypropionic acid is polymerized in the presence of the polyol, a branched prepolymer is prepared, and this branched prepolymer has a large molecular weight, so that impurities can be easily removed in the subsequent water washing process. Specifically, in the case of 3-hydroxypropionic acid, dehydration occurs and the monomer reaction terminal is converted to a vinyl group, terminating the polymerization, and/or a low molecular weight cyclic structure is formed during the polycondensation process, making it difficult to improve the molecular weight, but the preparation method according to the one embodiment can use the polyol in the polymerization process to prepare a branched prepolymer with a large molecular weight.

The polyol is not particularly limited in type as long as it is used for preparing the 3-hydroxypropionic acid fermentation broth, but for example, it may comprise one or more selected from the group consisting of glycerol, pentaerythritol, 4-arm-poly(ethyleneglycol)n=2~10, di(trimethylolpropane), di(pentaerythritol), tri(pentaerythritol), xylitol, sorbitol, inositol, cholic acid, β-cyclodextrin, and tetrahydroxyperylene.

In addition, during the polymerization of 3-hydroxypropionic acid, the polyol may be included in an amount of 0.01 wt% or more and 10.0 wt% or less based on 100 wt% of 3-hydroxypropionic acid, for example, 0.03 wt% or more, 0.05 wt% or more, or 0.08 wt% or more, or 10.0 wt% or less, 9.0 wt% or less, 8.0 wt% or less, 7.0 wt% or less, 6.0 wt% or less, 5.0 wt% or less, 4.0 wt% or less, 3.0 wt% or less, 2.0 wt% or less, 1.5 wt% or less, 1.0 wt% or less, or 0.8 wt% or less. Within the above content range, a branched prepolymer that can achieve the desired objective can be prepared, and if the polyol is included in an excessively small amount, a branched prepolymer may not be prepared, and if the polyol is included in an excessively large amount, impurities may be generated during polymerization or a gelation phenomenon may occur.

The polymerization of 3-hydroxypropionic acid in the presence of the polyol may be carried out at a temperature of 70°C or more and 100°C or less, and for example, it may be carried out at a polymerization temperature of 75°C or more, 78°C or more, 80°C or more, 83°C or more, or 85°C or more, or at a polymerization temperature of 98°C or less, 95°C or less, 93°C or less, or 90°C or less. If the polymerization temperature is too low, the polymerization may hardly proceed, resulting in the preparation of a 3-hydroxypropionic acid prepolymer with a low molecular weight, and if the polymerization temperature is too high, dehydration of 3-hydroxypropionic acid may occur, converting the monomer reaction terminal to a vinyl group, so that a 3-hydroxypropionic acid prepolymer may not be prepared.

The polymerization may be carried out under vacuum. At this time, the vacuum state means a pressure state lower than atmospheric pressure, and may mean, for example, a pressure state of 500 torr or less. Although not particularly limited, the polymerization may be carried out at a pressure of 100 torr or less, 50 torr or less, 10 torr or less, 1 torr or less, or 0.1 torr or less.

The polymerization may be performed for 1 to 70 hours. Specifically, the polymerization performed within the

above-described vacuum state and temperature range may be carried out for 2 hours or more, 3 hours or more, 4 hours or more, 5 hours or more, 8 hours or more, 10 hours or more, 15 hours or more, or 20 hours or more, and for 60 hours or less, 55 hours or less, 50 hours or less, 45 hours or less, 40 hours or less, 35 hours or less, 30 hours or less, or 25 hours or less. At this time, the polymerization may be carried out for a time during which side reactions are suppressed and a sufficient yield can be secured.

In addition, the polymerization may be carried out without a catalyst, or the polymerization may be carried out in the presence of a catalyst. The use of a catalyst is advantageous for promoting the polymerization reaction and for suppressing the formation of cyclic oligomers during the polymerization process. The type of catalyst is not particularly limited as long as it does not interfere with the progress of the polymerization reaction or the achievement of the technical problem of the present application. The catalyst that can be used may be, for example, an acid catalyst or a tin-based catalyst. The acid catalyst may be, for example, a sulfonic acid-based catalyst or include the same, and examples of the sulfonic acid-based catalyst include p-toluenesulfonic acid, m-xylene-4-sulfonic acid, 2-mesitylenesulfonic acid, and/or p-xylene-2-sulfonic acid. In addition, as the tin-based catalyst, for example, $SnCl_2$ or $Sn(oct)_2$ may be used.

The catalyst may be used in a predetermined content range. For example, the polymerization may be performed using the catalyst in a content of 0.001 to 1.0 mol% with respect to the 3-hydroxypropionic acid. Specifically, the content of the catalyst may be 0.01 mol% or more, 0.05 mol% or more, or 0.1 mol% or more, and the upper limit thereof may be, for example, 0.9 mol% or less, 0.8 mol% or less, 0.7 mol% or less, 0.6 mol% or less, or 0.5 mol% or less. When the catalyst is used within the above-described content range, it may be more advantageous for promoting polymerization and suppressing the formation of cyclic oligomers.

In the method for preparing branched 3-hydroxypropionic acid prepolymer according to the one embodiment, the branched 3-hydroxypropionic acid prepolymer may be represented by the following Formula 1.

[Formula 1] $R\text{-}[A\text{-}(B)n\text{-}C]_k$

In the Formula 1,

R is a functional group with a valence of 3 or more derived from polyol,
A is a direct bond or a linking group derived from ether, sulfide, ester, thioester, ketone, sulfoxide, sulfone, sulfonate ester, amine, amide, imine, imide, or urethane,
B is a substituent represented by the following Formula 2 or Formula 3,

[Formula 2]

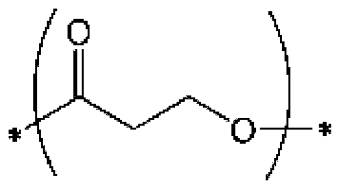

[Formula 3]

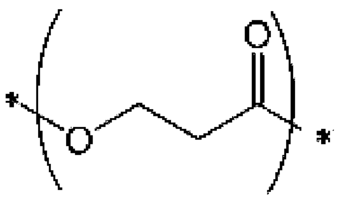

* is a part connected to A, k is an integer of 3 or more, n is an integer of 1 to 700, and
C is a substituent represented by the following Formula 4 or Formula 5.

[Formula 4]

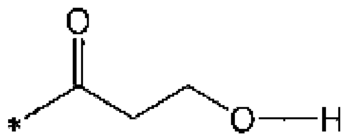

[Formula 5]

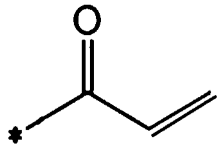

.

At this time, branched means a polymer of a monomer in which each functional group is 3 or more or 4 or more, and the R part in Formula 1 is defined as a branched structure.

For example, the branched structure may mean structures such as

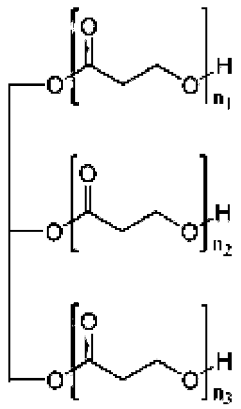

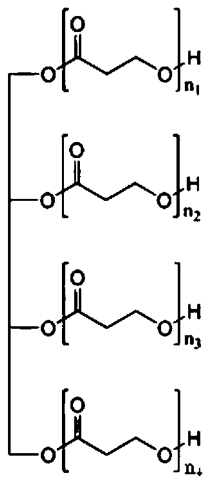

,

and the like, but is not limited thereto. In each branched unit, n may independently have any integer value from 1 to 700.

In one example, k of Formula 1 may be an integer of 3 or more, 4 or more, 5 or more, 6 or more, 7 or more, or 8 or more. Although not particularly limited, k of Formula 1 may be 20 or less, 18 or less, 16 or less, 14 or less, 12 or less, 10 or less, 8 or less, or 6 or less.

In one example, R may be a linking group with a valence of 3 or more derived from substituted or unsubstituted $C_{1-60}$ alkyl, substituted or unsubstituted $C_{3-60}$ cycloalkyl, substituted or unsubstituted $C_{6-60}$ aryl, or substituted or unsubstituted $C_{2-60}$ heteroaryl containing one or more of N, O, and S. At this time, at least one of the carbon atoms of the alkyl, cycloalkyl, aryl, and heteroaryl may be substituted or unsubstituted with at least one heteroatom selected from the group consisting of N, O, and S, or a carbonyl.

In addition, in the branched 3-hydroxypropionic acid prepolymer, the ratio of the number of Formula 5 to the total of Formula 4 and Formula 5, that is, the vinyl group content at branched terminal , may be 5 % or more, 7 % or more, 10 % or more, 13 % or more, 15 % or more, 20 % or more, 25 % or more, or 30 % or more, or 100 % or less, 95 % or less, 90 % or less, 80 % or less, 75 % or less, 72 % or less, 70 % or less, 65 % or less, 60 % or less, or 56 % or less. Since the branched 3-hydroxypropionic acid prepolymer exhibits the above-mentioned vinyl group content at branched terminal, the content of vinyl groups exhibiting hydrophobicity can be increased instead of hydrophilic hydroxyl groups.

Furthermore, as 3-hydroxypropionic acid is polymerized and the molecular weight increases, the branched 3-hydroxypropionic acid prepolymer exhibits hydrophobicity as the ratio of hydrophilic carboxyl groups and hydroxyl groups decreases, and as the hydroxyl groups at the terminal of this hydrophobic prepolymer are also converted to vinyl groups, it

can exhibit stronger hydrophobicity. Such a prepolymer may be easy to remove inorganic and organic impurities when washed in the washing process described below.

Meanwhile, the ratio of the number of Formula 5 to the total of Formula 4 and Formula 5 (vinyl group content at branched terminal) can be obtained by measuring the vinyl group structure at the prepolymer chain end using $^1$H-NMR.

Specifically, the vinyl group content at branched terminal can be calculated by the following Equation 1 by measuring the C-H peak value of the vinyl group of Formula 5 at the branched terminal of each prepolymer (①) and the terminal beta C-H peak value of Formula 4 (②) using $^1$H-NMR.

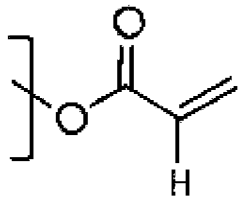

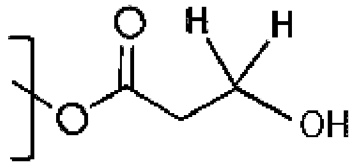

**① Vinyl C-H (peak 6.38)** **② Terminal Beta C-H (peak 3.86)**

[Equation 1]

vinyl group content at branched terminal (%) = ①/(1+②/2) X 100

In Equation 1, ① is the C-H peak value of the vinyl group of Formula 5 at the branched terminal of the branched 3-hydroxypropionic acid prepolymer, and ② is the terminal beta C-H peak value of Formula 4 at the branched terminal of the 3-hydroxypropionic acid prepolymer.

At this time, in the Formula 4 or 5, * may be a part connected to B.

In addition, the branched 3-hydroxypropionic acid prepolymer satisfies an acid value of 150 meq/kg or less. Specifically, the acid value of the branched prepolymer may be, for example, 140 meq/kg or less, 135 meq/kg or less, 130 meq/kg or less, 125 meq/kg or less, 120 meq/kg or less, 115 meq/kg or less, 110 meq/kg or less, 105 meq/kg or less, 100 meq/kg or less, 95 meq/kg or less, 90 meq/kg or less, 85 meq/kg or less, 80 meq/kg or less, 75 meq/kg or less, 70 meq/kg or less, 65 meq/kg or less, 60 meq/kg or less, 55 meq/kg or less, 50 meq/kg or less, 45 meq/kg or less, 40 meq/kg or less, 35 meq/kg or less, or 30 meq/kg or less. Within the above acid value range, the branched prepolymer can have a stable state, and the acid value can be measured by titration with 0.02 N Potassium methoxide solution as a titration solution.

In addition, the branched 3-hydroxypropionic acid prepolymer may have a weight average molecular weight of 1,800 or more and 90,000 or less, and may be 2,000 or more, 3,000 or more, 5,000 or more, 7,000 or more, 9,000 or more, or 10,000 or more, or 85,000 or less, 80,000 or less, 75,000 or less, 70,000 or less, 65,000 or less, 60,000 or less, 50,000 or less, 40,000 or less, 30,000 or less, or 20,000 or less.

In addition, the branched 3-hydroxypropionic acid prepolymer may have a number average molecular weight of 1,200 or more and 80,000 or less, and may be 1,500 or more, 1,800 or more, 2,000 or more, 3,000 or more, 5,000 or more, 7,000 or more, or 8,000 or more, or 75,000 or less, 70,000 or less, 65,000 or less, 60,000 or less, 55,000 or less, 50,000 or less, 45,000 or less, 40,000 or less, 35,000 or less, 30,000 or less, 25,000 or less, 20,000 or less, 15,000 or less, or 10,000 or less.

If the weight average molecular weight and number average molecular weight of the branched 3-hydroxypropionic acid prepolymer are too low, the prepolymer may exhibit hydrophilicity, making it difficult to remove impurities contained in the prepolymer in the washing process, and in particular, it may be difficult to recover the prepolymer from which impurities have been removed during layer separation after the washing process. If the weight average molecular weight and number average molecular weight are too high, coagulation of the prepolymer may easily occur during layer separation after the subsequent washing process, making process control difficult, and it may be difficult to prepare acrylic acid in the subsequent pyrolysis process.

In addition, the branched prepolymer may have a polydispersity index (PDI) within the range of 1.0 to 13.0. Specifically, the polydispersity index (PDI) of the prepolymer may be, for example, 1.2 or more, 1.4 or more, 1.6 or more, 1.8 or more, 2.0 or more, or 3.0 or more, and its upper limit may be, for example, 12.0 or less, 11.5 or less, 11.0 or less, 10.5 or less, 10.0 or less, 9.5 or less, 9.0 or less, 8.5 or less, 8.0 or less, 7.5 or less, 6.5 or less, 6.0 or less, 5.5 or less, 5.0 or less, or 4.0 or less.

In addition, when polymerizing the 3-hydroxypropionic acid, a linear 3-hydroxypropionic acid prepolymer may

also be prepared in addition to the branched 3-hydroxypropionic acid prepolymer, but based on 100 wt% of the total of the branched and linear prepolymers, the branched prepolymer may be included in an amount of 70 wt% or more, 80 wt% or more, 85 wt% or more, 90 wt% or more, 95 wt% or more, or 100 wt%.

Since the branched 3-hydroxypropionic acid prepolymer has a significantly larger molecular weight than the linear 3-hydroxypropionic acid prepolymer and exhibits hydrophobicity, impurities contained in the prepolymer can be easily removed in the washing process.

The method for preparing branched 3-hydroxypropionic acid prepolymer according to the one embodiment may comprise a step of washing the branched 3-hydroxypropionic acid prepolymer.

The washing process is a simple and low-cost process used to remove impurities contained in a target substance with a hydrophilic solvent such as water, and although it is difficult to remove impurities from the monomer 3-hydroxypropionic acid by washing, the branched 3-hydroxypropionic acid prepolymer prepared by the preparation method according to the one embodiment exhibits hydrophobicity, so impurities such as inorganic ions can be easily removed through washing.

Accordingly, if the washing of the branched 3-hydroxypropionic acid prepolymer is not performed, the prepolymer may contain a large amount of impurities, particularly hydrophilic inorganic impurities, which may lower the purity of the prepolymer, and when the prepolymer is later pyrolyzed to be converted into other monomers such as acrylic acid, the purity of the monomer is also lowered, and the time required for pyrolysis and purification may become excessive due to impurities.

For example, impurities can be removed by mixing the branched 3-hydroxypropionic acid prepolymer with a washing solution, and when impurities are removed by mixing with a washing solution while the prepolymer is in a flowable state, the purity of the finally recovered prepolymer can be higher.

In addition, after mixing or centrifuging the liquid prepolymer and the washing solution, a high-purity prepolymer can be recovered by layer separation into an oily phase (or lower layer) containing the prepolymer and an aqueous phase (or upper layer) containing the washing solution.

In addition, the washing may be carried out at a temperature of 60°C or more and 90°C or less and for a time of 1 minute or more and 30 minutes or less, and the washing temperature may be, for example, 63°C or more, 65°C or more, 67°C or more, 70°C or more, or 72°C or more, or at a temperature of 88°C or less, 87°C or less, or 86°C or less. If the washing temperature is too low, the prepolymer may not exhibit flowability, resulting in low impurity removal efficiency, and if the washing temperature is too high, the prepolymer may be hydrolyzed, causing loss of 3-hydroxypropionic acid.

In addition, the washing may be carried out for a time of 3 minutes or more, 5 minutes or more, 7 minutes or more, or 10 minutes or more, or 25 minutes or less, 20 minutes or less, or 15 minutes or less. If the washing time is too short, impurity removal may not be achieved, and if the washing time is too long, the prepolymer may be hydrolyzed, shortening its length and causing loss of 3-hydroxypropionic acid.

The washing may be performed with a washing solution comprising water and/or a basic compound. The washing solution may be, for example, aqueous ammonia, and the basic compound is not particularly limited as long as it is a basic compound that does not contain metal ions, but examples include quaternary ammonium compounds, amines, and the like.

Examples of the quaternary ammonium compound include tetramethylammonium hydroxide (TMAH), trimethyl-2-hydroxyethylammonium hydroxide (choline), tetraethylammonium hydroxide, tetrapropylammonium hydroxide, tetrabutylammonium hydroxide, trimethylphenylammonium hydroxide, benzyltrimethylammonium hydroxide, and the like.

The amine may be a primary aliphatic amine, a secondary aliphatic amine, a tertiary aliphatic amine, or a cycloaliphatic amine, and for example, the primary aliphatic amine may be monoethanolamine, ethylenediamine, 2-(2-aminoethoxy)ethanol, 2-(2-aminoethylamino)ethanol, diethylenetriamine, triethylenetetramine, etc., the secondary aliphatic amine may be diethanolamine, N-methylaminoethanol, N-hydroxyethylaminoethanol, dipropylamine, 2-ethylaminoethanol, etc., the tertiary aliphatic amine may be triethanolamine, dimethylaminoethanol, ethyldiethanolamine, etc., and the cycloaliphatic amine may be cyclopentylamine, cyclohexylamine, etc.

The content of the basic compound contained in the washing solution may be included in an amount of 1 wt% or more, 3 wt% or more, 5 wt% or more, 7 wt% or more, 8 wt% or more, or 10 wt% or more, and 30 wt% or less, 25 wt% or less, 20 wt% or less, 15 wt% or less, or 10 wt% or less, based on 100 wt% of the total washing solution.

In addition, during the washing, the washing solution may be used in a weight of 1.0 times or more and 30.0 times or less with respect to the weight of the prepolymer, and for example, it may be used in a weight of 1.2 times or more, 1.4 times or more, 1.5 times or more, 2.0 times or more, or 3.0 times or more, and 25.0 times or less, 20.0 times or less, 15.0 times or less, or 10.0 times or less.

In addition, the purity of the prepolymer recovered after the washing process may be 90% or more, 93% or more, 95% or more, 98% or more, 99% or more, or 100%.

According to another embodiment of the present invention, there is provided a method for preparing acrylic acid, comprising a step of fermenting a strain capable of producing 3-hydroxypropionic acid in the presence of a polyol to

prepare a fermentation broth comprising the polyol and 3-hydroxypropionic acid, a step of polymerizing the 3-hydroxypropionic acid in the presence of the polyol to prepare a branched 3-hydroxypropionic acid prepolymer, and a step of pyrolyzing the branched 3-hydroxypropionic acid prepolymer to prepare acrylic acid.

In the method for preparing acrylic acid, the 'step of fermenting a strain capable of producing 3-hydroxypropionic acid in the presence of a polyol to prepare a fermentation broth comprising the polyol and 3-hydroxypropionic acid, a step of polymerizing the 3-hydroxypropionic acid in the presence of the polyol to prepare a branched 3-hydroxypropionic acid prepolymer' is the same as the above-mentioned method for preparing branched 3-hydroxypropionic acid prepolymer, and like the preparation method, it may further comprise a step of separating the strain capable of producing 3-hydroxypropionic acid; a step of generating and separating metal salt crystals by adding an acid to the fermentation broth comprising the polyol and 3-hydroxypropionic acid; and/or a step of washing the branched 3-hydroxypropionic acid prepolymer.

The method for preparing acrylic acid according to the other embodiment comprises a step of pyrolyzing the branched 3-hydroxypropionic acid prepolymer to prepare acrylic acid.

The acrylic acid is prepared by pyrolyzing a prepolymer obtained by polymerizing 3-hydroxypropionic acid prepared by a biological process, which is environmentally friendly and economical, and the acrylic acid can be recycled as a bio-superabsorbent polymer (SAP) or a bio-acrylate.

The pyrolysis may be carried out at a temperature of 170°C or more and 250°C or less, for example, 180°C or more, 185°C or more, 190°C or more, or 195°C or more, and 240°C or less, 230°C or less, 225°C or less, 220°C or less, 215°C or less, or 210°C or less. If the pyrolysis temperature is too low, the pyrolysis of the branched 3-hydroxypropionic acid prepolymer may not be achieved, and if the pyrolysis temperature is too high, the pyrolysis process operating cost may increase or a large amount of unexpected impurities may be generated.

The acrylic acid may have a content of bio-based carbon measured by the ASTM 6866-21 standard of 80 wt% or more, for example, 85 wt% or more, 90 wt% or more, 95 wt% or more, or 100 wt%. The content of bio-based carbon means the content of bio-based carbon relative to the total carbon content contained in the acrylic acid, and the larger this value, the more it may correspond to an environmentally friendly compound. As a measurement method, for example, the carbon atoms contained in the compound to be measured are made into graphite or carbon dioxide gas form and measured with a mass spectrometer, or measured by liquid scintillation spectroscopy. At this time, along with the mass spectrometer, etc., an accelerator for separating $^{14}C$ ions from $^{12}C$ ions can be used together to separate the two isotopes and measure the content and content ratio with a mass spectrometer.

## ADVANTAGEOUS EFFECTS

According to the present invention, it is possible to provide a method for preparing a high-molecular-weight and high-purity branched prepolymer from low-purity, unpurified 3-hydroxypropionic acid efficiently and at a low process cost, and preparing bio-based acrylic acid by pyrolyzing it.

## DETAILED DESCRIPTION OF THE EMBODIMENTS

Hereinafter, the present disclosure will be described in more detail with reference to examples. However, the following examples are provided only for the purpose of illustrating the present disclosure, and thus the scope of the present disclosure is not limited thereto.

### Example 1

### (1) Production and purification of 3-hydroxypropionic acid fermentation broth

A recombinant vector was prepared by introducing genes encoding Glycerol dehydratase and Aldehyde dehydrogenase, which are known to produce 3-hydroxypropionic acid (3-HP) using glycerol as a substrate. The prepared recombinant vector was introduced into an E. coli W3110 strain to construct a 3-hydroxypropionic acid-producing strain.

More specifically, a pCDF_J23101_dhaB_gdrAB_J23100_aldH_btuR vector obtained by cloning the BtuR gene encoding adenosyltransferase into a plasmid pCDF containing the gene (dhaB) encoding glycerol dehydratase, the gene (aldH) encoding aldehyde dehydrogenase, and the gene (gdrAB) encoding glycerol dehydratase reactivase was introduced into a W3110 strain (KCCM 40219) by electroporation using an electroporation device (Bio-Rad, Gene Pulser Xcell) to construct a 3-hydroxypropionic acid-producing strain. The process of constructing the 3-hydroxypropionic acid-producing strain of this Preparative Example 1 and the vectors, primers, and enzymes used were carried out with reference to Example 1 of Korean Laid-open Patent Publication No. 10-2020-0051375.

The prepared 3-hydroxypropionic acid-producing strain was cultured by fermentation in a 5L fermenter at 35°C using crude glycerol as a carbon source to produce 3-hydroxypropionic acid. To prevent a decrease in pH due to the

production of 3-hydroxypropionic acid, an alkali metal salt, calcium hydroxide ($Ca(OH)_2$), was added to maintain the pH at neutral during fermentation.

After fermentation culture, the strain was removed by centrifugation (4000 rpm, 10 min, 4 °C), and the fermentation broth was titrated to pH 2 using sulfuric acid ($H_2SO_4$). Thereafter, after evaporation and concentration at 45°C and 20 torr, gypsum ($Ca(SO)_4$) crystals generated during the sulfuric acid titration process were removed through solid-liquid separation. Thereafter, 0.2 wt% of sulfuric acid was added to proceed with the protonation of 3-hydroxypropionic acid, and water was distilled for 4 hours at 80°C and 60 torr to prepare 3-hydroxypropionic acid. At this time, the 3-hydroxypropionic acid comprises glycerol at 0.08 wt% based on 100 wt% of 3-hydroxypropionic acid.

### (2) Preparation of prepolymer

80 g of the 3-hydroxypropionic acid containing glycerol and 250 mg of a p-toluenesulfonic acid (p-TSA) catalyst were added to a 100 ml glass reactor, and a polymerization reaction was carried out at a temperature of 90°C and F.V. (Full Vacuum) conditions for 24 hours to prepare a branched 3-hydroxypropionic acid prepolymer (weight average molecular weight: 5,000, number average molecular weight: 3,000).

### (3) Washing of prepolymer

A washing solution was prepared by mixing 1000 ml of water and 100 ml of aqueous ammonia, and the 3-hydroxypropionic acid prepolymer was washed twice for 10 minutes at a speed of 150 rpm at a temperature of 85°C. At this time, the washing solution was used in a weight 1.5 times that of the 3-hydroxypropionic acid prepolymer. Thereafter, the lower layer containing the washed prepolymer was recovered by phase separation (layer separation), and dried in an oven at a temperature of 40°C for 20 hours to finally recover the dried 3-hydroxypropionic acid prepolymer (purity: 99% (excluding water), moisture content: <5 wt%).

### Example 2

A 3-hydroxypropionic acid prepolymer was prepared in the same manner as in Example 1, except that the prepolymer was not washed.

### Reference Example 1

A 3-hydroxypropionic acid-producing strain was cultured in the same manner as in Example 1 to produce 3-hydroxypropionic acid, and the strain was removed by centrifugation (4000 rpm, 10 min, 4 °C). Primary fermentation broth purification (primary purification) was carried out using activated carbon. Specifically, activated carbon was added to the fermentation broth from which the strains had been removed by centrifugation, mixed well, and then centrifuged again to separate the activated carbon. Thereafter, the fermentation broth from which the activated carbon was separated was filtered through a 0.7 $\mu$m filter paper with a vacuum pump to purify the 3-hydroxypropionic acid fermentation broth.

The concentration of 3-hydroxypropionic acid in the fermentation broth that had undergone the primary purification was at a level of 50 to 100 g/L, and the fermentation broth was concentrated to a concentration of 600 g/L using a rotary evaporator (50°C, 50 mbar) to prepare a concentrate, and twice the volume of ethanol of the concentrate was added and stirred at room temperature (300 rpm) to generate $Ca(3HP)_2$ crystals. At this time, the concentration of the alkali metal salt in the concentrate was 493.3 g/L (based on $Ca(OH)_2$). The generated crystals were subjected to washing 3 times using ethanol (EtOH) and dried in an oven at 50°C to finally recover the crystals.

An aqueous solution containing 600 g/L of the recovered $Ca(3HP)_2$ crystals was prepared and stirred at a temperature of 25°C and 350 rpm for 10 minutes. To 77 mL of the aqueous solution, 37 mL of a 5M sulfuric acid solution was added at a uniform rate over 5 minutes and further stirred for 30 minutes to form a slurry comprising $CaSO_4$ precipitates and 3-hydroxypropionic acid. In order to separate the $CaSO_4$ precipitates, filtration was performed using a filtration flask and a vacuum pump, and a filtrate containing 3-hydroxypropionic acid was obtained.

## Evaluation

### 1. Measurement of inorganic impurities content

The content of inorganic impurities contained in the 3-hydroxypropionic acid prepolymer of Example 1 finally recovered after washing and the 3-hydroxypropionic acid of Reference Example 1 was measured using ICP-OES (Inductively Coupled Plasma - Optical Emission Spectrometry), and the results are shown in Table 1 below. Meanwhile, cases not detected were indicated as N.D. (Not Detected).

Specifically, about 0.1 g of a sample was dissolved by adding 0.5 mL of nitric acid and shaking overnight at room temperature. To promote the reaction of the sample, a small amount of hydrogen peroxide was added to dissolve it, and when the sample was clearly and completely dissolved, it was diluted to 10 mL with tertiary ultrapure water, and undissolved components were removed with a 0.45 μm PTFE filter, and then the remaining filtrate was injected into an ICP-OES instrument (AVIO 500, Perkin Elmer) to perform component analysis.

<ICP-OES analysis conditions>

RF Power: 1300W
Torch Height: 15 mm
Plasma gas flow rate: 15 L/min
Sample gas flow rate: 0.8 L/min
Auxiliary gas flow rate: 0.20 L/min
Pump speed: 1.5 mL/min

**2. Yield measurement**

The yield of the 3-hydroxypropionic acid prepolymer of Example 1 finally recovered after washing was measured through weight measurement and GPC/FT-RI, and the yield of the 3-hydroxypropionic acid of Reference Example 1 was measured through concentration measurement by HPLC (high performance liquid chromatography), and the results are shown in Table 1 below.

Specifically, the measurement by GPC used a 150C ALC/GPC (manufactured and sold by Waters Assoc. Co., USA), and the measurement by FT-IR was performed by dissolving 20 to 30 mg of a sample in 15 ml of trichlorobenzene having a temperature of 140°C, and applying 500 to 1,000 μl of the obtained solution to an FT-IR device (Perkin-Elmer 1760X, Perkin Elmer Cetus, USA).

In addition, for the HPLC analysis, an HPLC system from Waters (e2695 separations module, 2998 photodiode array detector) was used, and for the column, a Capcellpak C18 (4.6mm ID X 50mm L, particle size: 3 μm) from Shiseido was used at 40°C. As the mobile phase, acetonitrile (ACN, for HPLC, J.T. Baker) and ultrapure water (MILLIPORE, Mili-Q, 18.2MΩ) were degassed, and a concentration gradient was given for the ACN/H2O ratio from 20/80 (v/v) to 60/40 (v/v) for 20 minutes, and the flow rate was set to 1 mL/min. 10 μL of the analysis sample was injected, and the chromatogram was measured at 280nm using a photodiode array detector (PDA).

[TABLE 1]

| | Inorganic impurities content (ppm) | | | | | | Yield (%) |
|---|---|---|---|---|---|---|---|
| | $Na^+$ | $K^+$ | $Ca^{2+}$ | $Cl^-$ | $S^-$ | $P^-$ | |
| Example 1 | 1090 | 237 | 18 | 914 | 2150 | N.D. | 93 |
| Example 2 | 23082 | 3772 | 353 | 7031 | 27537 | 30 | 95 |
| Reference Example 1 | 2140 | 356 | 389 | 3200 | 2720 | 27 | 92 |

According to Table 1, it was confirmed that in Examples 1 and 2, 3-hydroxypropionic acid was polymerized in the presence of the glycerol used in the 3-hydroxypropionic acid production process, and a branched 3-hydroxypropionic acid prepolymer (weight average molecular weight: 5,000, number average molecular weight: 3,000) exhibiting hydrophobicity was prepared. In addition, it was confirmed that Examples 1 and 2 have a high yield because less 3-hydroxypropionic acid is lost due to fewer purification steps compared to the conventional purification process, and the yield is higher than in Reference Example 1, where an activated carbon treatment process, an electrodialysis process, etc., are performed.

In particular, it was confirmed that Example 1, in which the prepolymer washing process was performed, contained a small amount of inorganic impurities similar to Reference Example 1 and showed an equivalent level of yield with only a polymerization process and a water washing process, even though it did not undergo two activated carbon treatment processes, an electrodialysis process, and a purification process using an ion exchange resin like Reference Example 1. Meanwhile, since the sulfur ion (S-) is due to the catalyst used during the polymerization process, it is predicted that the content of sulfur ions can be controlled by controlling the amount of catalyst used in the polymerization process.

**Example 3: Preparation of acrylic acid**

10 g of the 3-hydroxypropionic acid prepolymer prepared and washed in Example 1 was added to a flask and heated to 200°C, and then the acrylic acid that distilled out was recovered. After the reaction was completed, a total of 3 g of acrylic acid was recovered (yield: 30%).

**Example 4: Preparation of acrylic acid**

10 g of the 3-hydroxypropionic acid prepolymer prepared and washed in Example 1 and 10 mg of hydroquinone (polymerization inhibitor) were added to a flask and heated to 200°C, and then the acrylic acid that distilled out was recovered. After the reaction was completed, a total of 7 g of acrylic acid was recovered (yield: 70%).

**Example 5: Preparation of acrylic acid**

10 g of the 3-hydroxypropionic acid prepolymer prepared and washed in Example 1 and 0.3 g of N,N,N',N",N"-Pentamethyldiethylenetriamine (high-boiling point organic base) were added to a flask and heated to 200°C, and then the acrylic acid that distilled out was recovered. After the reaction was completed, a total of 8.5 g of acrylic acid was recovered (yield: 85%).

**Example 6: Preparation of acrylic acid**

10 g of the 3-hydroxypropionic acid prepolymer prepared and washed in Example 1 and 15 mg of sodium hydroxide (NaOH, inorganic base) were added to a flask and heated to 200°C, and then the acrylic acid that distilled out was recovered. After the reaction was completed, a total of 5 g of acrylic acid was recovered (yield: 50%).

## Claims

1. A method for preparing branched 3-hydroxypropionic acid prepolymer, comprising:

   a step of fermenting a strain capable of producing 3-hydroxypropionic acid in the presence of a polyol to prepare a fermentation broth comprising the polyol and 3-hydroxypropionic acid: and
   a step of polymerizing the 3-hydroxypropionic acid in the presence of the polyol to prepare a branched 3-hydroxypropionic acid prepolymer.

2. The method for preparing branched 3-hydroxypropionic acid prepolymer of Claim 1,
   wherein the polymerization is carried out at a temperature of 70°C or more and 100°C or less.

3. The method for preparing branched 3-hydroxypropionic acid prepolymer of Claim 1,
   wherein the polyol comprises one or more selected from the group consisting of glycerol, pentaerythritol, 4-arm-poly(ethyleneglycol)n=2~10, di(trimethylolpropane), di(pentaerythritol), tri(pentaerythritol), xylitol, sorbitol, inositol, cholic acid, β-cyclodextrin and tetrahydroxyperylene.

4. The method for preparing branched 3-hydroxypropionic acid prepolymer Claim 1,
   wherein the polyol is comprised in an amount of 0.01 wt% or more and 10.0 wt% or less based on 100 wt% of the 3-hydroxypropionic acid during the polymerization.

5. The method for preparing branched 3-hydroxypropionic acid prepolymer of Claim 1,
   further comprising a step of washing the branched 3-hydroxypropionic acid prepolymer.

6. The method for preparing branched 3-hydroxypropionic acid prepolymer of Claim 5,
   wherein the washing is carried out with a washing solution comprising water and/or a basic compound.

7. The method for preparing branched 3-hydroxypropionic acid prepolymer of Claim 5,
   wherein the washing is carried out at a temperature of 60°C or more and 90°C or less and for a time of 1 minute or more and 30 minutes or less.

8. The method for preparing branched 3-hydroxypropionic acid prepolymer of Claim 1,

further comprising a step of separating the strain capable of producing 3-hydroxypropionic acid; and/or a step of generating and separating metal salt crystals by adding an acid to the fermentation broth comprising the polyol and 3-hydroxypropionic acid, after the step of fermenting a strain capable of producing 3-hydroxypropionic acid in the presence of a polyol to prepare a fermentation broth comprising the polyol and 3-hydroxypropionic acid.

**9.** The method for preparing branched 3-hydroxypropionic acid prepolymer of Claim 1,

wherein the branched 3-hydroxypropionic acid prepolymer is represented by the following Formula 1:

[Formula 1] $R\text{-}[A\text{-}(B)n\text{-}C]_k$

in the Formula 1,
R is a functional group with a valence of 3 or more derived from polyol,
A is a direct bond or a linking group derived from ether, sulfide, ester, thioester, ketone, sulfoxide, sulfone, sulfonate ester, amine, amide, imine, imide or urethane,
B is a substituent represented by the following Formula 2 or Formula 3,

[Formula 2]

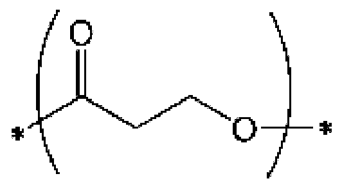

[Formula 3]

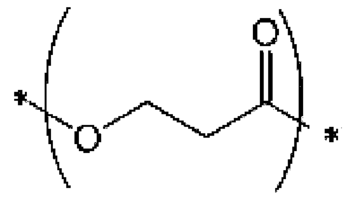

* is a part connected to A, k is an integer of 3 or more, n is an integer of 1 to 700, and
C is a substituent represented by the following Formula 4 or Formula 5.

[Formula 4]

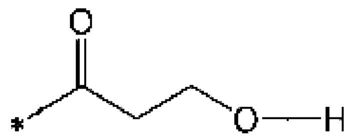

[Formula 5]

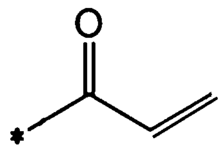

**10.** The method for preparing branched 3-hydroxypropionic acid prepolymer of Claim 9,

wherein a ratio of the number of Formula 5 to a total of Formula 4 and Formula 5 is 5% or more.

**11.** The method for preparing branched 3-hydroxypropionic acid prepolymer of Claim 1,
wherein the branched 3-hydroxypropionic acid prepolymer has a weight average molecular weight of 1,800 or more and 90,000 or less.

**12.** A method for preparing acrylic acid, comprising:

a step of fermenting a strain capable of producing 3-hydroxypropionic acid in the presence of a polyol to prepare a fermentation broth comprising the polyol and 3-hydroxypropionic acid;
a step of polymerizing the 3-hydroxypropionic acid in the presence of the polyol to prepare a branched 3-hydroxypropionic acid prepolymer; and
a step of pyrolyzing the branched 3-hydroxypropionic acid prepolymer to prepare acrylic acid.

**13.** The method for preparing acrylic acid of Claim 12,
wherein the pyrolysis is carried out at a temperature of 170°C or more and 250°C or less.

**14.** The method for preparing acrylic acid of Claim 12,
wherein the acrylic acid has a content of bio-based carbon of 80 wt% or more as measured by the ASTM 6866-21 standard.

## INTERNATIONAL SEARCH REPORT

International application No. **PCT/KR2025/007352**

**A. CLASSIFICATION OF SUBJECT MATTER**

**C12P 7/52**(2006.01)i; **C07C 51/09**(2006.01)i; **C07C 51/377**(2006.01)i; **C07C 57/04**(2006.01)i

According to International Patent Classification (IPC) or to both national classification and IPC

**B. FIELDS SEARCHED**

Minimum documentation searched (classification system followed by classification symbols)

C12P 7/52(2006.01); B01D 61/44(2006.01); B01J 31/02(2006.01); C07C 51/09(2006.01); C07C 51/47(2006.01); C07C 51/573(2006.01); C07C 57/04(2006.01); C07C 67/03(2006.01); C08G 63/06(2006.01); C08G 63/78(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 분지형 3-하이드록시프로피온산 프리폴리머 (branched 3-hydroxypropionic acid prepolymer), 폴리올 (polyol), 아크릴산 (acrylic acid)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2023-0079986 A (LG CHEM, LTD.) 07 June 2023 (2023-06-07)<br>paragraphs [0032], [0093], [0103] | 1-14 |
| Y | KR 10-2024-0064557 A (LG CHEM, LTD.) 13 May 2024 (2024-05-13)<br>claims 1, 3, 5; paragraphs [0095], [0096], [0099], [0114] | 1-14 |
| Y | KR 10-2023-0166952 A (LG CHEM, LTD.) 07 December 2023 (2023-12-07)<br>claims 1, 4, 11 | 12-14 |
| Y | KR 10-2015-0032579 A (BASF SE) 26 March 2015 (2015-03-26)<br>claim 1 | 12 |
| Y | KR 10-2018-0085746 A (BASF SE) 27 July 2018 (2018-07-27)<br>claims 1-12; paragraph [0007] | 12 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 August 2025** | **27 August 2025** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**
**Information on patent family members**

International application No.
**PCT/KR2025/007352**

| Patent document cited in search report | Publication date (day/month/year) | Patent family member(s) | Publication date (day/month/year) |
|---|---|---|---|
| KR 10-2023-0079986 A | 07 June 2023 | None | |
| KR 10-2024-0064557 A | 13 May 2024 | CN 119156411 A | 17 December 2024 |
| | | EP 4509542 A1 | 19 February 2025 |
| | | JP 2025-516008 A | 23 May 2025 |
| | | KR 10-2024-0138255 A | 20 September 2024 |
| | | WO 2024-096665 A1 | 10 May 2024 |
| KR 10-2023-0166952 A | 07 December 2023 | CN 117794889 A | 29 March 2024 |
| | | US 2024-0383837 A1 | 21 November 2024 |
| | | WO 2023-234688 A1 | 07 December 2023 |
| KR 10-2015-0032579 A | 26 March 2015 | AU 2013-292147 A1 | 05 February 2015 |
| | | AU 2013-292147 B1 | 16 February 2017 |
| | | BR 112015000892 A2 | 27 June 2017 |
| | | CN 104619678 A | 13 May 2015 |
| | | CN 104619678 B | 22 February 2017 |
| | | DE 102012212424 A1 | 16 January 2014 |
| | | EP 2872476 A1 | 20 May 2015 |
| | | JP 2015-522595 A | 06 August 2015 |
| | | JP 6238984 B2 | 29 November 2017 |
| | | RU 2015104903 A | 27 August 2016 |
| | | RU 2640591 C2 | 10 January 2018 |
| | | SG 11201408547 QA | 27 February 2015 |
| | | TW 201410647 A | 16 March 2014 |
| | | TW I593672 B | 01 August 2017 |
| | | US 2014-0018574 A1 | 16 January 2014 |
| | | WO 2014-012856 A1 | 23 January 2014 |
| | | ZA 201501000 B | 25 May 2016 |
| KR 10-2018-0085746 A | 27 July 2018 | BR 112018009801 A2 | 06 November 2018 |
| | | BR 112018009801 A8 | 26 February 2019 |
| | | CN 108350158 A | 31 July 2018 |
| | | EP 3377554 A1 | 26 September 2018 |
| | | JP 2018-536712 A | 13 December 2018 |
| | | US 2018-0327345 A1 | 15 November 2018 |
| | | WO 2017-085120 A1 | 26 May 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020240071883
- KR 1020250070312
- KR 1020200051375